# EUROPEAN PATENT SPECIFICATION

(11) **EP 1 661 641 B1**
(45) Date of publication and mention of the grant of the patent: **19.05.2010**
(21) Application number: 05380031.4
(22) Date of filing: 18.02.2005
(51) Int. Cl.: B22C 9/10

(54) **Core for obtaining a thermostatic tap**
Kern für termostatische Mischbatterie
Noyau pour faucet thermostatique

(30) Priority: 24.11.2004 ES 200402675
(43) Date of publication of application: 31.05.2006
(73) Proprietor: Caspro, S.A., E-08759 Vallirana - Barcelona (ES)
(72) Inventor: Tres Casas, Daniel, 08759 Vallirana (Barcelona) (ES)
(74) Representative: Ungria Lopez, Javier

(56) References cited:
- EP-A- 1 186 982
- GB-A- 2 076 127

## Description

### OBJECT OF THE INVENTION

As stated in the title of this specification, this invention concerns a core for obtaining a thermostatic tap, said core presenting the particular feature of being materialised in a single body of sand, on the basis of which the corresponding tap can be obtained with its inlet, outlets and internal ducts.

The object of the invention is to reduce the time and costs of manufacturing thermostatic taps, on the basis of previously achieving a core by injection of sand into the respective moulding box, resulting following the stripping of a single solid body with the appropriate shapes for obtaining the thermostatic tap body by moulding by means of pouring of the molten metal into a mould.

### BACKGROUND OF THE INVENTION

As is known, in obtaining certain metallic pieces, and specifically bodies for thermostatic taps, sand cores are used which are obtained by injection of sand into a moulding box, with a binder or resin being added to the sand in order to achieve a body that can stay as a single piece and thereby permit the respective body of a thermostatic tap to be obtained, to which will then be added the controls and other accessories necessary in the tap.

As is also known, in obtaining the body of a thermostatic tap, several cores are used which are achieved independently in different moulding boxes, by sand injection, in such a way that those cores define specific parts of the body of the tap to obtain and they are then joined later on or arranged in the appropriate manner in the mould on which the metal is poured in order to achieve the body of the tap under consideration.

As is evident, due to having several cores for obtaining the body of the tap, it is necessary to create as many sand moulding boxes, which implies an economic cost.

Moreover, also to be added on are the costs implied by the time taken in joining the cores required in obtaining the body of the tap and thereby the need for labour in order to carry out all the operations and or/handling required in the process.

Document EP 1 186 982 A2 describes a thermostatic tap trying to avoid the heating of the principal body by means of circulating the hot water from a mixing cartridge until the exit (14), using a duct (13) that remains externally covered by a cold water duct (12), thus achieving to minimize the hot touch of the body of the tap.

Document GB 2 076 127 A describe a system for thermostatically controlling of the temperature by means of mixing valves for cold and hot water and by means of a stainless bimetallic thermostatic spiral (146).

### DESCRIPTION OF THE INVENTION

The invention is based on the fact that the body of the thermostatic tap is defined starting from a single sand core, the latter presenting the particular feature of being constituted by means of a single-piece body of cylindrical general configuration, with individual cylindrical sections being shaped at the ends which are those which, in the body of the tap obtained from that core, establish the housings where the controls for the tap are going to be fitted, in other words, the thermostatic temperature control and the opening/closing control for the flow, regulation and distribution thereof.

From those cylindrical sections there emerge individual shapes by way of bridges, which in the body of the tap will establish the inlets for hot and cold water, constituting means of reinforcement for preventing the breakage of certain parts of plateaus established in the actual core, which are extended longitudinally in order to form ducts for the travel of hot and cold water when obtaining the body of the tap. The said bridges are extended between the said cylindrical parts and ends of the body of the core and the actual parts of plateaus referred to. The hot water and the cold water are mixed in a duct established in the proximity of one of the inlets, which will be the one for the hot water as will be commented upon further below, with that mixing duct being obtained by means of an annular projection provided in the body of the core, whose location lies between the end cylindrical section of the respective core and the projection in which the plateau close to that end is prolonged.

In the body of the core, the end part where the plateau of shorter length is planned to be, will, in the body of the tap obtained, correspond to the inlet for hot water and mixing with cold water, and it will also correspond to the location of the regulating control for the temperature of the water, while the opposite end part will, in the body of the tap obtained, correspond to the inlet for cold water and to the location of the control for opening/closing of the flow of water and distribution thereof towards the desired outlet.

In the body of the core, the central part thereof presents a transverse circular opening for the location of threaded elements which, in obtaining the body of the tap, have to establish individual outlets which connect with individual ducts, via which and by means of turning the opening/closing and distribution control in one direction or the other, will direct the water towards one or the other of those outlets.

The said distribution ducts are carried out by means of axial drill-holes made on the central part of the body of the tap which has already been obtained on the basis of the said core.

The body of the tap obtained on the basis of the core described here presents the regulating control for temperature in correspondence with the end where the inlet is established for hot water and mixing with cold water, which will determine a minimum travel for the hot water and therefore a maximum travel for the cold water which comes from the opposite end, which means that the body of the tap is heated solely in the short section where the hot water ducts are located, which are the ones with less length since, as was said, the cold water circulates through the duct that runs along the body of the tap.

By means of the core of the invention, a three-way (one inlet and two outlets) thermostatic tap body is obtained, with major advantages being achieved as a consequence of the core in question being achieved in a single-piece body of sand, with the following being able to be cited as the most notable advantages:
- Reduction of costs, due to the core being created in a single piece instead of in several as is conventionally required.
- Elimination of the processes of manufacture of several independent cores and of the operation of joining them together.
- Reduction in labour.
- Increase in production due to elimination of phases or steps in the manufacturing chain of the cores, such as joining of different independent cores in order to form or achieve the compound core.

### BRIEF DESCRIPTION OF THE FIGURES

In order to complete the description that is going to be made forthwith, and with the aim of aiding a better understanding of the features of the invention, this specification is accompanied by a set of drawings on the basis of which the innovations and advantages of the sand core for obtaining a thermostatic tap will be more easily understood.
Figure 1 shows a longitudinal view of the core forming the object of the invention.
Figure 2 shows a perspective view of the same core represented in the previous figure.
Figure 3 shows another perspective view, in this case from a different angle, of the core represented in the previous figures.
**Figure 4** shows a view in longitudinal section of the body of the thermostatic tap obtained from the core represented in the previous figures.
Figure 5 shows a view corresponding to one of the ends of the body of the thermostatic tap represented in the previous figure.
Figure 6 shows a view in transverse section of the body of the thermostatic tap represented figures 4 and 5.

### DESCRIPTION OF AN EMBODIMENT OF THE INVENTION

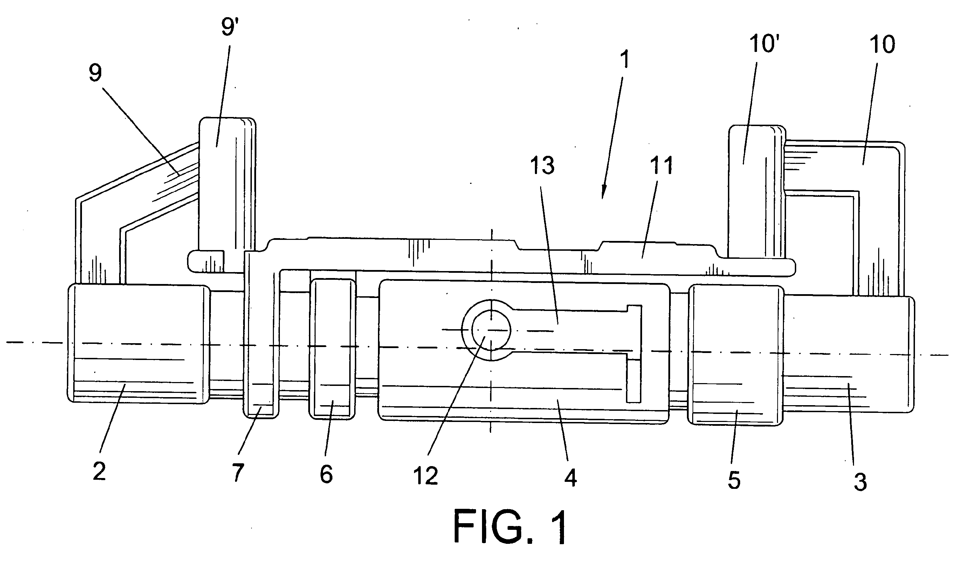

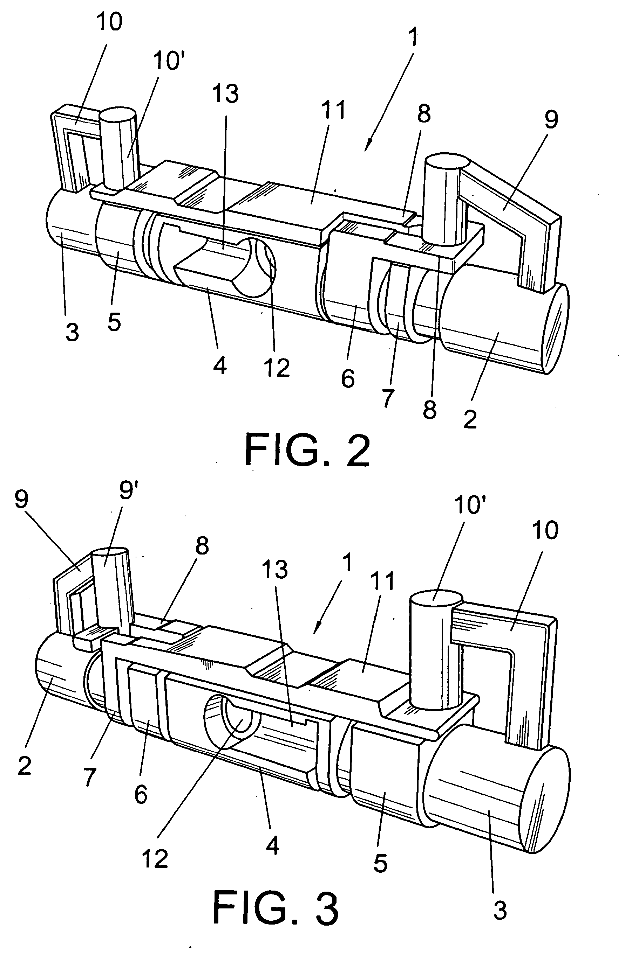

Looking at figures 1, 2 and 3, the sand core 1 can be seen, which presents the form of an elongated body of cylindrical general shape, with two end parts of cylindrical configuration and which are referenced with numbers 2 and 3, a central part 4, and between this and the cylindrical part 3, a projected part 5, while between said central part 4 and the cylindrical end part 2 are established two annular projections 6 and 7, the first of them as a prolongation of a plateau part 8 between which and the end cylindrical part 2 a kind of bridge 9 is provided, which is repeated, though with another configuration according to reference 10 at the opposite end, in other words between the cylindrical part 3 and the other plateau part 11 of greater length than the previous one, since the latter is extended from that cylindrical end section 3 as far as the annular projection 6 provided in the proximity of the opposite part. The plateau part 8 referred to in the first place occupies a short section of this end part, and it can be said that both plateau parts 8-11 form a rectangular and longitudinal projection between the bridges 9 and 10, which, as well as establishing the inlets in obtaining the body of the tap, also determine means of reinforcement in order to prevent breakage of the plateau parts 8 and 11, which have to shape the ducts for the circulation of hot and cold water.

In the central part 4 of the body 1 of the core there exists a transverse opening 12 of circular section, widening towards the ends, while collaterally to this, on both sides of that central part 4, there exists a depression 13, whose function, as with the rest of the characteristics, will be explained forthwith.

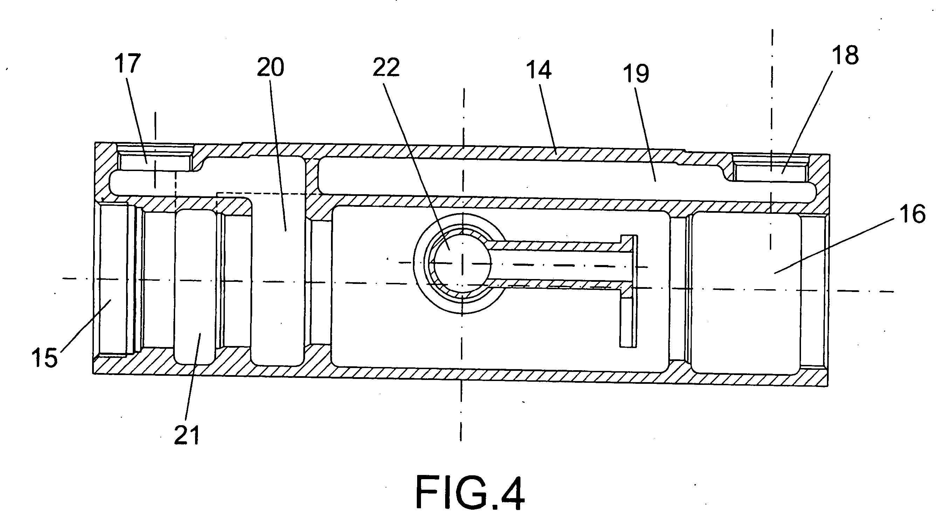

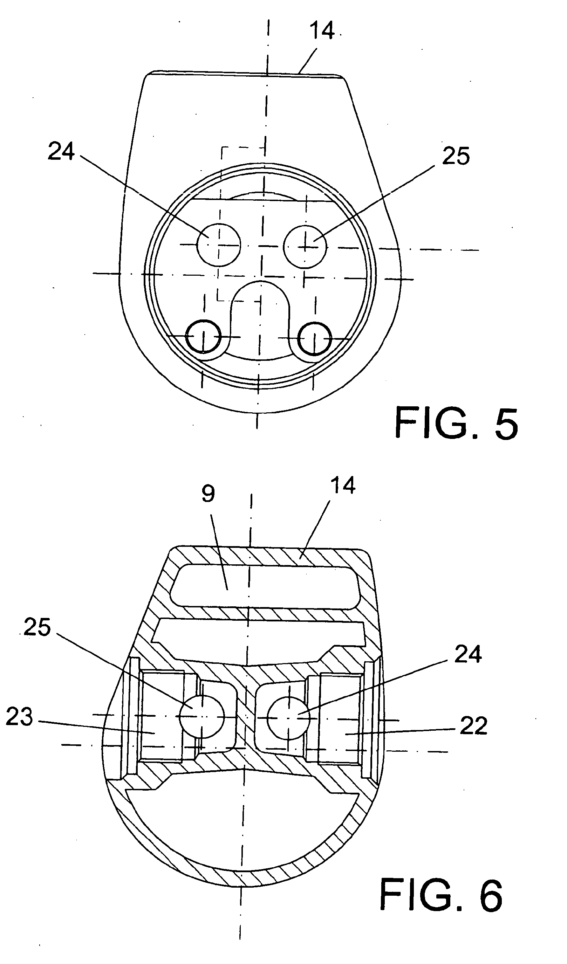

By means of the core thus constituted and achieved in a moulding box by injection of sand with a binder or resin, a thermostatic tap is obtained whose body is that referenced with number 14 in figures 4, 5 and 6, all this in such a way that the end parts and cylinders 2 and 3 of core 1 establish, in the obtaining of the body of the tap 14, housings 15 and 16 for the fitting of controls for temperature regulation for the water and for opening/closing and distribution of the flow of water towards the desired outlet, respectively.

For their part, the cylindrical and radial shapes 9' and 10' of the bridges 9 and 10 establish in the body of the tap 14 the inlet 17 for hot water and the inlet 18 for cold water.

In terms of the section or plateau part 11 of the core 1, in obtaining the body of the tap 14, the duct 19 for cold water will be established as far as reaching the duct 20 provided in the inlet zone for hot water, and whose duct 19 connects with the inlet cavity or chamber 21 for cold water, with the hot water inlet being established in that duct 20, said duct 20 being determined by means of the plateau 8 and the annular projection 6 in which the former is prolonged, while the chamber 21 for cold water is established in obtaining the body of the tap 14 by means of the projection 7 of that end part of the core 1.

Likewise established in that body of the tap 14 are the two outlets 22 and 23 which are obtained as a consequence of the fact that, provided in the transverse opening 12 of the body of the core 1 are threaded elements which will determine those outlets 22 and 23, which connect with the ducts 24 and 25 made on the solid centre of the interior of the body of the tap 14 once this has been obtained, in other words, those ducts 24 and 25 are effected directly on the body of the tap 14 without being obtained by means of the core 1, as is the case with all the other ducts and holes.

That central part or metal blocks of the body of the tap 14 are formed as a consequence of the cavities 13 in the core 1.

## Claims

1. **CORE FOR OBTAINING A THERMOSTATIC TAP,** obtained by injection of sand in a moulding box, **characterised in that** the core comprises
- a single-piece body (1), on the basis of which, by means of pouring of metal into the respective mould, the body of the thermostatic tap (14) is obtained without any additional accessories, said single-piece body of the core (1) presenting a cylindrical general configuration with
- two cylindrical end sections (2 and 3) by means of which individual housings (15 and 16) are established in the body of the resulting tap (14) for the fitting of the controls for temperature regulation of the water and for opening/closing, distribution and regulation of the flow of that water;
- a central part (4) being presented between said cylindrical end sections (2 and 3), provided with
- a circular and transverse opening (12) and
- individual lateral depressions (13) opposing each other, by means of which individual outlets (22 and 23) are established in the body of the resulting tap (14) and their connection with distribution ducts (24 and 25) for the water towards said outlets (22 and 23);
- provision having been made so that, between said central part (4) of the core body and the cylindrical end sections (2 and 3), individual intermediate zones are established, between which
- a projecting longitudinal plateau is provided with a plateau section (11) of larger length and
- a plateau section (8) of lesser length, with the feature that between each plateau section (8 and 11) and the nearest cylindrical section (2 and 3)
- a bridge is defined at an angle (9 and 10), with inlets for hot water (17) and for cold water (18) being established in the body of the resulting tap (14), while the projecting plateau parts (8 and 11) establish the ducts (19 and 20) for the circulation of cold water and hot water, furthermore incorporating
- an annular projection (7) in which the part of the plateau (8) joined to the bridge (9) is prolonged, in order to establish in the body of the resulting tap (14) an opening for cold water towards the duct (20) for hot water, the mixing of hot and cold water being internally carried out at the thermostatic cartridge that is fitted, with this mixing duct (20) being established by means of
- an annular projection (6) provided between the projection (7) and the respective central section (4) of that end of the body of the core (1).

2. **CORE FOR OBTAINING A THERMOSTATIC TAP**, according to claim 1, **characterised in that** the cylindrical end section (2) establishing the housing (15) of the resulting tap (14) corresponds to that for fitting the regulating control for the water temperature, while the opposite end, cylindrical section (3) establishes the housing (16) of the resulting tap (14) that corresponds to that for fitting the control for opening/closing and distribution and regulation of the flow of water towards the desired outlet (22 or 23), with the projecting plateau section (11) corresponding to the duct (19) for the circulation of cold water of the resulting tap (14), the bridge (10) of that end being by means of which the inlet for cold water (18) is established in the body of the resulting tap (14).

3. **CORE FOR OBTAINING A THERMOSTATIC TAP**, according to the preceding claims, **characterised in that** the central section (4) of the body of the core (1), being affected by the transverse opening (12) and the two lateral depressions (13), establishes a solid core with two holes (22 and 23) in the resulting body of the tap (14), corresponding to the outlets, said holes connecting in the body of the tap (14) with individual axial ducts (24 and 25) for water distribution, said holes (24 and 25) being made subsequent to the resulting body of the tap (14) itself.

## Patentansprüche

1. Kern zur Erzeugung einer Thermostatarmatur, der durch das Injizieren von Sand in einen Formkasten gewonnen wird, **dadurch gekennzeichnet, dass** der Kern Folgendes umfasst:
• einen einstückigen Körper (1), auf dessen Grundlage durch das Gießen von Metall in die entsprechende Form ohne zusätzliche Komponenten der Körper der Thermostatarmatur (14) gewonnen wird, wobei der einstückige Körper des Kerns (1) eine zylindrische Allgemeingestaltung hat, mit
• zwei zylindrischen Endabschnitten (2 und 3), durch die im Körper der entstandenen Thermostatarmatur (14) zwei einzelne Gehäuse (15 und 16) eingerichtet sind, in denen die Steuerungen für die Temperaturregulierung des Wassers und für das Öffnen/Schließen, die Verteilung und die Regulierung des Stroms dieses Wassers angeordnet sind;
• einem Mittelteil (4), der zwischen den zylindrischen Endabschnitten (2 und 3) angebracht ist, ausgestattet mit
• einer runden und in die Querrichtung verlaufenden Öffnung (12) und
• einzelnen seitlichen Vertiefungen (13), die einander gegenüber liegen, und durch die einzelne Auslässe (22 und 23) im Körper der entstandenen Thermostatarmatur (14) eingerichtet sind, und ihre Verbindung mit Verteilkanälen (24 und 25), um das Wasser an die Auslässe (22 und 23) zu leiten,
• wobei vorgesehen ist, dass zwischen dem Mittelteil (4) des Körpers des Kerns und den zylindrischen Endabschnitten (2 und 3) einzelne Zwischenzonen eingerichtet sind, zwischen denen
• eine vorstehende, in die Längsrichtung verlaufende Plattform mit einem Plattformteil (11) von größerer Länge und
• einem Plattformteil (8) von kürzerer Länge vorgesehen ist, mit dem Merkmal, dass zwischen jedem Plattformteil (8 und 11) und dem nächsten zylindrischen Abschnitt (2 und 3)
• eine Brücke (9 und 10) in einem Winkel definiert ist, wobei Einlässe für heißes Wasser (17) und für kaltes Wasser (18) im Körper der entstandenen Armatur (14) eingerichtet sind, während die vorstehenden Plattformteile (8 und 11) die Kanäle (19 und 20) für die Zirkulation des kalten Wassers und des heißen Wassers definieren, die des Weiteren Folgendes einschließen:
• einen ringförmigen Vorsprung (7), in dem der Teil der Plattform (8), der mit der Brücke (9) verbunden ist, verlängert ist, um im Körper der entstandenen Armatur (14) eine Öffnung für kaltes Wasser in den Kanal (20) für heißes Wasser zu bilden, wobei das Mischen von heißen und kaltem Wasser im Inneren der eingesetzten Thermostatkartusche erfolgt, wobei dieser Mischkanal (20) durch
• einen ringförmigen Vorsprung (6) gebildet ist, der zwischen dem Vorsprung (7) und dem entsprechenden Mittelteil (4) an jenem Ende des Körpers des Kerns (1) bereitgestellt ist.

2. Kern zur Erzeugung einer Thermostatarmatur nach Anspruch 1, **dadurch gekennzeichnet, dass** der zylindrische Endabschnitt (2), der das Gehäuse (15) für die entstandene Armatur (14) bildet, jenem entspricht, in dem die Steuerung für die Regulierung der Wassertemperatur angeordnet ist, während der gegenüber liegende zylindrische Endabschnitt (3) das Gehäuse (16) der entstandenen Armatur (14) bildet, das jenem für das Anordnen der Steuerung für Öffnen/Schließen und die Verteilung und Regulierung des Wasserstroms an den erwünschten Auslass (22 oder 23) entspricht, wobei der vorstehende Plattformteil (11) dem Kanal (19) für die Zirkulation von kaltem Wasser der entstandenen Armatur (14) entspricht, und wobei der Einlass für kaltes Wasser (18) durch die Brücke (10) dieses Endes im Körper der entstandenen Armatur (14) eingerichtet ist.

3. Kern zur Erzeugung einer Thermostatarmatur nach den vorhergehenden Ansprüchen, **dadurch gekennzeichnet, dass** der Mittelteil (4) des Körpers des Kerns (1), in dem die in die Querrichtung verlaufende Öffnung (12) und die zwei seitlichen Vertiefungen (13) gebildet sind, im entstandenen Körper der Armatur (14) einen festen Kern mit zwei Löchern (22 und 23) bildet, die den Auslässen entsprechen, wobei sich die Löcher im Körper der Armatur (14) mit einzelnen Axialkanälen (24 und 25) für die Verteilung des Wassers verbinden, und wobei die Löcher (24 und 25) nach dem entstandenen Körper der Armatur (14) selbst hergestellt werden.

## Revendications

1. Noyau pour robinet thermostatique, obtenu par injection de sable dans un boîtier de moulage, **caractérisé en ce que** le noyau comprend :
- un corps (1) en une pièce à partir duquel, le corps du robinet thermostatique (14) est obtenu sans utiliser aucun accessoire supplémentaire en versant du métal dans le moule concerné, ledit corps en une pièce du noyau (1) présentant une configuration globalement cylindrique qui présente
- deux parties d'extrémité cylindriques (2 et 3) au moyen desquelles des logements individuels (15 et 16) destinés à placer les commandes de régulation de température de l'eau, d'ouverture et de fermeture, de répartition et de régulation de l'écoulement de cette eau sont formés dans le corps du robinet (14) ainsi obtenu,
- une partie centrale (4) prévue entre lesdites parties d'extrémité cylindriques (2 et 3) et présentant
- une ouverture transversale circulaire (12) et
- des creux latéraux (13) distincts, situés l'un en face de l'autre et au moyen desquels des sorties distinctes (22 et 23) et leur raccordement à des conduits de distribution (24 et 25) de l'eau en direction desdites sorties (22 et 23) sont formées dans le corps du robinet (14) ainsi obtenu,
- des moyens étant prévus pour former entre ladite partie centrale (4) du corps de noyau et les parties cylindriques d'extrémité (2 et 3) des zones intermédiaires séparées entre lesquelles
- un plateau longitudinal en saillie est doté d'une partie de plateau (11) de plus grande longueur et
- une partie en plateau (8) de longueur plus courte, qui présente la caractéristique qu'entre chaque partie en plateau (8 et 11) et la partie cylindrique (2 et 3) la plus proche,
- un pont est défini sous un angle (9 et 10), des entrées pour l'eau chaude (17) et pour l'eau froide (18) sont formées dans le corps du robinet (14) ainsi obtenu, les parties de plateau en saillie (8 et 11) formant les conduits (19 et 20) pour la circulation de l'eau froide et de l'eau chaude, et comprenant en outre
- une saillie annulaire (7) dans laquelle la partie du plateau (8) reliée au pont (9) se prolonge pour établir dans le corps du robinet (14) ainsi obtenu une ouverture pour l'eau froide en direction du conduit (20) pour l'eau chaude, le mélange de l'eau chaude et de l'eau froide étant assuré à l'intérieur sur la cartouche thermostatique qui y est placée, ce conduit de mélange (20) étant formé au moyen de
- une saillie annulaire (6) prévue entre la saillie (7) et la partie centrale (4) respective de chaque extrémité du corps du noyau (1).

2. Noyau pour robinet thermostatique selon la revendication 1, **caractérisé en ce que** la partie cylindrique d'extrémité (2) qui forme le logement (15) du robinet (14) ainsi obtenu correspond à celle prévue pour installer le contrôle de régulation de la température d'eau, tandis que la partie cylindrique (3) de l'extrémité opposée forme le logement (16) du robinet (14) ainsi obtenu qui correspond à celui prévu pour l'installation de la commande d'ouverture et de fermeture, de distribution et de régulation de l'écoulement d'eau en direction de la sortie (22 ou 23) souhaitée, la partie (11) en plateau en saillie correspondant au conduit (19) de recirculation d'eau froide du robinet (14) ainsi obtenu, le pont (10) de cette extrémité permettant d'établir l'entrée d'eau froide (18) dans le corps du robinet (14) ainsi obtenu.

3. Noyau pour robinet thermostatique selon les revendications précédentes, **caractérisé en ce que** la partie centrale (4) du corps du noyau (1) affectée par l'ouverture transversale (12) et par les deux creux latéraux (13) forme dans le corps du robinet (14) ainsi obtenu un noyau plein doté de deux trous (22 et 23) qui correspondent aux sorties, lesdits trous étant reliés dans le corps du robinet (14) à des conduits axiaux (24 et 25) distincts prévus pour la distribution de l'eau, lesdits trous (24 et 25) étant réalisés après le corps du robinet (14) proprement dit.
